(19) Europäisches Patentamt
European Patent Office
Office européen des brevets

(11) EP 0 614 081 B1

(12) **EUROPEAN PATENT SPECIFICATION**

(45) Date of publication and mention of the grant of the patent:
**12.07.2000 Bulletin 2000/28**

(51) Int. Cl.[7]: **G01N 27/02**

(21) Application number: **93116848.8**

(22) Date of filing: **19.10.1993**

# (54) A method and a system for measuring the concentration of a substance in a fluid, and the use thereof

Methode und System zur Messung der Konzentration einer Substanz in einem Fluid, und deren Anwendung

Méthode et système pour la mesure de la concentration d'une substance dans un fluide, et son utilisation

(84) Designated Contracting States:
**DE ES FR GB IT NL SE**

(30) Priority: **01.03.1993 IT TO930145**

(43) Date of publication of application:
**07.09.1994 Bulletin 1994/36**

(73) Proprietor: **BELLCO S.p.A.**
**41037 Mirandola (Modena) (IT)**

(72) Inventors:
- **Caputo, Giuseppe**
  **I-85040 Castelluccio Inferiore (Potenza) (IT)**
- **Della Ciana, Leopoldo**
  **i-48022 Lugo (Ravenna) (IT)**
- **Sacco, Silvio**
  **I-13013 Coggiola (Vercelli) (IT)**

(74) Representative:
**Rambelli, Paolo et al**
**c/o JACOBACCI & PERANI S.p.A.**
**Corso Regio Parco, 27**
**10152 Torino (IT)**

(56) References cited:
**DE-C- 3 900 119** **US-A- 3 839 154**

- **DATABASE WPI Week 8224, Derwent Publications Ltd., London, GB; AN 8249137 & JP-A-57 074 097 (YOKOGAWA ELECTRIC WKS KK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

Field of the invention

The present invention relates in general to the measurement of the concentration of a substance in a fluid.

The invention has been developed with particular attention to its possible use for determining the concentration of urea in biological fluids, for example, in dialysis equipment.

As will be explained further below, the invention is not limited to this possible use but is applicable, in general, to the measurement of the concentration in a fluid of any substance which can be converted into conversion products so as to bring about a change in the conductivity (the electrical conductivity) of the fluid carrying the substance.

Description of the prior art

In order to calculate the concentration of a substance in a fluid, possibly in the form of a continuous flow, it is generally known to correlate the variation of a certain parameter detectable by the measurement device, which differs from one case to another, with the variation in the concentration of the substance in the fluid under examination.

For example, known biosensors for determining urea concentration are based on the application of the potentiometric method, with electrodes having ion-selective membranes or with electrodes having gas-permeable membranes, or on the application of the thermometric method, on the application of the conductimetric method, or even on the application of the spectrophotometric method.

The relevant literature is quite extensive.

By way of reference, the following articles may be mentioned, for example:

- "An enzyme electrode for the substrate urea" by G.G. Guilbault and J.G. Montalvo, which appeared in the "Journal of the American Chemical Society", Vol. 92, No. 8, April 1970;
- "Urea coupled ammonia electrode for urease determination in blood serum" by M. Mascini and G.G. Guilbault, which appeared in "Analytical Chemistry", Vol. 49, No. 6, May 1977;
- "Enzyme urea biosensor based on a modified potentiometric pvc-nonactin membrane electrode for assay of urea in blood" by S.B. Butt and K. Camman, which appeared in "Analytical letters", Vol. 25, No. 9, September 1992;
- "Determination of urea with an ammonia gas-sensitive semiconductor device in combination with urease" by F. Winquist, A. Spetz and I. Lundström, which appeared in Analytica Chimica Acta 163, 1981; and

"Determination of creatinine by an ammonia-sensitive semiconductor structure and immobilized enzymes" by F. Winquist, A. Spetz and I. Lundström, which appeared in Analytical Chemistry, Vol. 58, 1986.

Guilbault and Montalvo described, in the article mentioned, a biosensor for urea prepared by immobilizing the urease enzyme in a polyacrylamide matrix on a nylon net. The net was then arranged on an ion-selective electrode responding to the ammonium ion. This biosensor (even in the subsequently perfected and improved versions) has various problems which limit its possible uses, particularly when it is to be used on-line on a dialysis machine.

A somewhat similar approach is disclosed, e.g., in DE-C-39 00 119, after which the preamble of Claim 1 was patterned. There, enymatic conversion is contemplated of the substance where concentration is to be determined.

In the first place, in fact, such arrangements require a preliminary calibration of the sensor with a known solution and concentration and various periodic calibrations to prevent the problem of the drift of the response over time; this inevitably increases the operations necessary on the part of the operator, whereas the tendency is to limit such operations as much as possible.

Moreover, this type of sensor necessitates slow flows: the main flow line of the liquid analyzed therefore has to be bypassed and an auxiliary pump used, consequently increasing costs.

Furthermore, these sensors have an intrinsic limitation, that is, they have poor selectivity for ammonium ions in the presence of potassium and sodium ions.

As can be seen from the article by Butt and Camman, this latter problem can be avoided with the use of a urea biosensor with a gas-permeable membrane which is sensitive, in the specific case, to ammonia. However, in addition to the fact that it does not solve the problems relating to calibration and flow, the electrode with a gas-permeable membrane has the disadvantage that it has slow response times and a slow return to the base line.

Other sensors provide for the use of MOS (metal-oxide semiconductor) devices such as, for example, those described by Winquist et al.. The MOS devices used in these sensors are capacitors the capacitance-voltage characteristics of which vary in dependence on the concentration of ammonia adsorbed on the surface of the metal. The ammonia detected is the reaction product of the enzymatic hydrolysis of urea by means of urease or of the hydrolysis of creatinine by means of creatinase (in which a 100% conversion of the substrate is assumed). A disadvantage of these sensors is represented by the interference of amines with low molecular weights and of hydrogen. The problems

relating to calibration, to small sample volumes and to the need for auxiliary accessories (pump, valve, stock of buffer), however, remain.

As regards the use of the thermometric method, the article "Determination of serum urea with an enzyme thermistor using immobilized urease" by B. Danielsson, K. Gadd, B. Mattiasson and K. Mosbach, which appeared in "Analytical letters", Vol. 9, No. 11, 1976, may be mentioned.

The thermometric biosensor described by Danielsson et al. measures the heat produced when urea passes through a column containing immobilized urease enzyme ; it has a wide measurement range and the linearity extends almost up to 200 mmol/l. This sensor operates with very slow flows and the sample is introduced in pulses by means of a three-way valve. The problems with this sensor are its high degree of sensitivity to flow variations, the long waiting time until a stable base line is obtained, and the need for calibration.

Examples of the use of the conductimetric method are provided by the articles "Conductivity method for determination of urea" by W.T. Chin and W. Kroontye, which appeared in "Analytical Chemistry", Vol. 33, No. 12, November 1961; "Application de la conductimetrie à l'étude des reactions enzymatiques - Système urée -uréase" (Application of conductimetry to the study of enzymatic reactions - the urea-urease system) by M. Hanss and A. Rey, which appeared in "Biochimica" and "Biophysica Acta" No. 227, 1971; "Conductimetric enzyme assay" by A.J. Lawrence, which appeared in "European Journal of Biochemistry", Vol. 18, No. 2, 1971; "Conductimetry in enzyme studies" by A.J. Lawrence and G.R. Moores, which appeared in "European Journal of Biochemistry", Vol. 24, No. 3, 1972; the article "A micro-electronic conductimetric biosensor" by D. Watson, P. Maynard, D.C. Cullen, R.S. Sephi, J. Breetle and C.R. Lowe, which appeard in "Biosensors", Vol. 3, No. 2, 1987/88; or the article "Continuous monitoring of urea in blood during dialysis" by P. Thavarungkul, H. Hakanson, O. Holst and B. Mattiasson, which appeared in "Biosensors & Bioelectronics", Vol. 6, 1991.

Lowe and his group developed a microelectronic conductimetric bio-sensor with a two-sensor configuration (one with and one without urease enzyme) which has a good correlation with the spectro-photometric reference method; it has a rather narrow measurement range, however, and requires the sample to be diluted with a buffer before measurement.

Another biosensor based on the conductimetric method has been developed by Mattiasson et al.. The system provides for the use of a sampling unit in combination with a conductimetric biosensor and also provides for an on-line arrangement for detecting the urea values during dialysis. Calibration with urea solutions of known concentration is required for this sensor. The whole system is quite complex. In fact it requires: a sampling unit, a blood heparinization line, an injector for the calibration or dialysis solution, a line for the dialysis buffer, the column with the immobilized enzyme, the conductivity monitoring cell. Since this cell is constituted by electrodes which are in direct contact with the dialysis liquid, however, it is subject to errors due to the adsorption of organic molecules and therefore requires frequent cleaning and calibration.

## Objects and summary of the invention

It is consequently of very great interest to develop a biosensor for the continuous determination of urea (and, in general, of other substances carried by a fluid) which can be integrated in equipment (such as a dialysis machine) with the ability to achieve on-line detection in real time.

For example, if reference is to be made (as will be done extensively below) to use in dialysis, this interest arises from the fact that the urea concentration, which is raised above the norm in a patient affected by kidney disease, decreases over time during dialysis and the dialysis may be defined as complete when the urea concentration values have been lowered so as to fall within the normal range. It is therefore very important to be aware of the progress of the urea concentration values in real time during the dialysis and to know that the value has returned within the norm at the actual moment when this occurs. This is in order to avoid a session being unnecessarily prolonged, the duration of the dialysis being determined, at the moment, on the basis of indirect data and being left to the discretion of the operator. Moreover, the dialysis patient may experience his condition in a less traumatic manner since he spends less time in specialized centres for each session.

The object of the present invention is therefore to respond to the interest expressed above and to do this - it is underlined once more - not with reference to the determination of urea alone but, as regards the determination of the concentration of a substance in a fluid, particularly in a flow, in general. According to the present invention, this object is achieved by virtue of a method having the further characteristics recited in the characterising portion of Claim 1. The invention also relates to a system for carrying out the method and to the use of the method for determining the concentrations of substances in fluids in equipment for purifying biological fluids such as, for example, blood.

In summary, in a typical embodiment, the present invention provides for the formation of a differential conductimetric circuit which is connected "on-line" in a flow line (which may be either a main flow line or, possibly, a branch or bypass flow line which, there appropriate, is activated selectively solely during the time intervals in which the monitoring is to be carried out) in which a fluid flow carries a substance the concentration of which is to be determined.

For example, the fluid in question may (as will be seen further below) be a liquid ultrafiltered within a dialysis process, in which the urea concentration is to be monitored.

Theoretical basis of the invention

In general terms, the present invention is based on the possibility of carrying out a total conversion of the substance the concentration of which is to be monitored into conversion products so as to bring about a change in the electrical conductivity of the fluid containing the conversion products.

Thus, if the electrical conductivity of the fluid containing the substance is measured before the conversion, so as to obtain a first electrical conductivity value, and the electrical conductivity of the fluid containing the products of the total conversion of the substance is then measured, so as to obtain a second conductivity value, it can be observed that the variation (typically the difference) between the electrical conductivity values determined before and after the total conversion is determined solely by the concentration of the substance in the fluid.

For example, in the case of the determination of urea carried out in an at least partially aqueous vector, it is possible to use a total enzymatic conversion by passing the fluid through a small column which is packed with excess urease, so as to convert all of the urea present in the sample into ions according to the reaction:

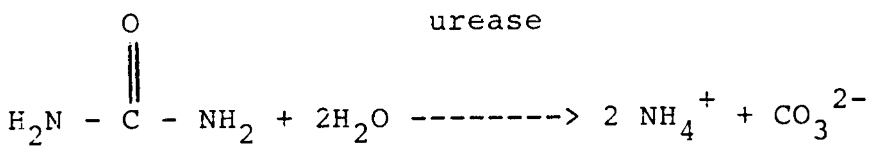


It can readily be seen that the electrical conductivity measurement carried out downstream of the total conversion reaction enables the base conductivity plus the conductivity due to the ions formed as a result of the conversion (hydrolysis in the present case) of the urea to be determined.

Naturally, in order to use this technique for measuring concentrations there must be a linear correlation between the conductivity measured and the concentration, at least in the measurement range. The basic relationship is as follows:

$$K = 10^3 \Lambda C \qquad (1)$$

in which K is the conductivity, C is the concentration, and $\Lambda$ is a proportionality constant known as the equivalent conductance.

$\Lambda$ may be evaluated by the direct measurement of the difference in the conductivity of the solution after complete enzymatic hydrolysis of a known quantity of urea. That is:

$$\Lambda = \frac{\Delta K}{C} \qquad (2)$$

An alternative method for evaluating $\Lambda$ consists of considering the hydrolysis products of urea: these are none other than the ions which make up the salt ammonium carbonate. It is thus possible to obtain $\Lambda$ directly by measuring the conductivity of ammonium carbonate solutions of known concentration.

As already stated, the principle described above with specific reference to a total enzymatic conversion of urea is suitable for use for determining the concentration of substances other than urea.

In general, in order to implement the invention, it is sufficient to be able to achieve a total enzymatic conversion of the substance of which the concentration is to be determined, into conversion products, so as to bring about a change in the electrical conductivity of the fluid carrying the substance. The desired concentration value can be derived by detecting the variation between the conductivity values determined before and after the conversion reaction.

In general, therefore, the solution according to the invention is suitable for use for the real-time and on-line measurement of the concentration of any substance for which it is possible to carry out a total enzymatic conversion by means of one or more corresponding enzymes.

By way of non-limiting example, the following conversion reactions may be mentioned:

---

creatinine amidohydrolase

creatinine + $H_2O$ ------------------------> creatine

creatine amidohydrolase

creatine + $H_2O$ ----------------------> sarcosine + urea

urease

urea + 2 $H_2O$ + $H^+$ ----------------------> $2NH_4^+$ + $HCO_3^-$

---

glucose oxidase

glucose + $O_2$ ---------------> $H_2O_2$ + gluconolactone

gluconolactone + $H_2O$ ------> gluconic acid + $H_2O_2$

gluconic acid + $H_2O$ ------> gluconate + $H^+$

---

barbiturase

barbiturate + $H_2O$ -----------> malonate + urea

urease

urea + $2H_2O$ + $H^+$ ------------> 2 $NH_4^+$ + $HCO_3^-$

---

arginase

L-arginine + $H_2O$ ------------> L-ornithine + urea

urease

urea + $2H_2O$ + $H^+$ -----------> 2 $NH_4^+$ + $HCO_3^-$

---

penicillinase

penicillin -------------> penicilloate + $H^+$

---

acetylcholinesterase

acetylcholine-------------------->choline + $CH_3COO^-$ + $H^+$

---

On the basis of the tests carried out by the Applicant, the enzymatic reaction was found particularly advantageous with regard to the fact that total conversion can be achieved extremely quickly (and hence so as to be able to provide a response in real time and on-line) within a reactor of simple structure and small dimensions and of correspondingly low cost.

Detailed description of an embodiment of the invention

The invention will now be described by way of non-limiting example with reference to the appended drawings, in which:

Figure 1 shows, in general, the hydraulic circuit of a haemodialysis system in which at least one sensor operating according to the invention may advantageously be incorporated,
Figure 2 shows the structure of parts of the sensor according to the invention in greater detail, and
Figures 3 to 7 are graphs showing results of the urea concentration measurements carried out according to the invention.

As indicated, Figure 1 shows the general structure of the hydraulic circuit of a dialysis machine operating according to the combined haemofiltration/haemodialysis principle.
The blood, which is taken from the patient P undergoing the treatment by a main circuit, generally indicated 1, is passed through a first peristaltic pump P1 which supplies it to a haemofilter HF and to a haemodialysis filter HD and is then re-admitted to the body of the patient P.
The fraction (the ultrafiltrate) removed in the haemofilter is restored upstream of the haemodialysis filter HD by means of an infusion solution taken from a respective reservoir R by means of a further peristaltic pump P2 which supplies the infusion solution to a pipe 2 which admits the infusion solution to the haemodialysis filter HD downstream of the haemofilter HF.
The two branches (intake and output) of the circuit which supplies the dialysis solution to the dialysis filter HD are indicated 3.
Finally, the line for discharging the ultrafiltrate from the haemofilter HF is indicated 4. A sensor 10, formed according to the invention, is connected in the line 4 and measures the concentration of urea in the ultrafiltrate coming from the haemofilter HF.
A non-return valve 5 is preferably associated with the sensor 10 for preventing any of the ultrafiltrate from returning towards the haemofilter HF.
In particular, it is known to make the pumps P1 and P2 subservient to a control or monitoring system, shown schematically in the form of two modules M1, M2 which are interdependent (for example, as a result of a contact operation effected by a unit such as a microprocessor or other processing unit) so as to regulate the haemofiltration and haemodialysis processes according to the requirements of the treatment in progress.
In general, the pump P1 regulates the flow-rate of the blood which is taken from and re-admitted to the patient in order to perform the purification treatment.
The pump P2 is regulated so that the reinfusion solution taken from the reservoir R is restored in the desired manner to the ultrafiltrate mass picked up by the filter HF.
The aforesaid control operations and other control and regulation operations are carried out in dependence on the various chemical/physical parameters which come into play during the treatment. In particular, one of these parameters is usually constituted by the urea concentration in the ultrafiltrate coming from the filter HF.
The availability of a sensor such as the sensor 10 which can provide the concentration of urea in the ultrafiltrate in real time and on-line makes the control of the purification process particularly effective and precise.
Naturally, sensors operating on the same principle as the sensor 10 described below may be inserted in a system such as the system of Figure 1 in order to monitor other concentration parameters (for example, for monitoring the concentration of creatinine in the ultrafiltrate coming from the haemofilter HF).
In this connection, it should be noted that - in order to monitor the concentration effectively, it is not in fact necessary for the whole of the flow containing the substance the concentration of which is to be monitored to pass through the sensor such as the sensor 10, as shown schematically in Figure 1.
In fact, it is possible to consider drawing off only a portion of the flow in which the concentration is to be monitored and passing through the sensor only that portion of the flow which has been drawn off. This possibility may also advantageously be used when it is desired to monitor the concentrations of two or more different substances in the same flow. For this purpose, for example, a corresponding number of branch flows may be drawn off from the main flow, and each may be passed through a respective sensor. Where appropriate, this may take place at different times and thus by drawing off a single branch flow and sending the flow to sensors of different types during successive time intervals. It is also possible to consider the positioning of several sensors fluidodynamically in series or in cascade, each sensor monitoring the concentration of a respective substance. Naturally, in order to be able to adopt this cascade arrangement, the total conversion carried out in each sensor must not affect the conversion mechanisms carried out in the other sensors situated downstream.
To proceed with a detailed examination of the structure of the sensor 10 (Figure 2), it can be noted that it is constituted essentially by three elements disposed in cascade in the direction of flow of the ultrafiltrate (from left to right

in Figure 2) through the sensor 10, that is:

- a first conductimetric sensor or probe 11,
- a conversion reactor 12 constituted, for example, by a small column of plastics material packed with an enzyme, such as urease (in the case of urea determination), previously bonded covalently to a substrate material such as, for example, particles of the material known by the trade name Eupergit C, and
- a further conductimetric sensor or probe 13.

The conductimetric probes 11 and 13 may be produced with the use of any solution known in the art for this purpose. However, it has been found particularly advantageous to use, within the scope of the invention, conductimetric probes such as those forming the subject of Italian patent IT-B-123 82 43. These conductimetric probes are produced by the company Tecnologie Dinamiche S.r.l., of Bologna (Italy).

By way of summary, it may be noted that a probe of this type is constituted essentially by a main tube 20 through which the liquid sample, the conductivity of which is to be measured, flows. In the probe, this pipe is divided into two parallel branches 20a, 20b which join together again at the output end of the probe (naturally, the direction of flow of the liquid sample through the probe is from left to right as shown by the arrows of Figure 2).

Two electromagnetic coils 21, 22 are disposed fluidodynamically upstream and downstream of each other (naturally still with reference to the direction of flow of the liquid sample through the probe) on the branch 20a. In practice, the conductimetric measurement is derived from a determination of the degree of coupling (magnetic induction) established between the two coils 21 and 22 by means of the liquid undergoing measurement. The main sources of errors for measurements of this type are fluctuations in the temperature of the liquid field. For this reason, the probes 11, 13 have thermistors 24 (or equivalent thermometric elements) which monitor the temperature of the liquid sample, preferably at the output ends of the probes 11, 13, and provide feedback to the control board 23 in order to put into effect a temperature compensation step.

Each of the conductimetric probes 11, 13 has an associated voltage measuring sensor 23a which emits, on a respective output line 11a, 13a, a signal indicative of the conductivity value measured in the liquid sample flowing through the respective probe. The conductivity signals obtained are sent (possibly after conversion into digital form by means of converter circuits, not shown, which may, however, be incorporated in the probes themselves) to a differentiator element or, in general, to a module 14 which is sensitive to the variation between the conductivity values detected by the probes 11 and 13. Alternatively, the two conductivity values detected by the probes 11 and 13 may also be detected separately, the task of determining their variation being left to a subsequent processing step.

As already stated (see the portion of the present description headed "Theoretical basis of the invention"), the difference between the values mentioned above constitutes an indication of the concentration of the substance which is being determined (for example, urea) in the flow passing through the sensor 10. Naturally, however, it is possible to consider the use of any other signal indicative of the variation between the two conductivity values (for example, their ratio, or the difference standardized with respect to one of the conductivity values measured, etc.) instead of the difference signal.

In any case, the signal coming from the module 14 which is sensitive to the variation between the two conductivity values measured by the probes 11, 13 can be transmitted to a unit 15 for presenting it externally, such as, for example, a display unit or a unit for recording it on a paper or informatics substrate.

The reactor 12 in which the enzymatic conversion of the urea into ammonium carbonate salt is carried out (according to the criteria recited extensively above), is preferably configured in the form of a small column of, for example, plastics material with respective input and output lines for the liquid sample which passes through the reactor 12 (again from left to right with reference to Figure 2).

Preferably, filter elements 30a, 31a are associated with the connecting lines of the reactor - constituted by pipes 30 and 31 (for input and output respectively) - and are constituted, for example, by membranes of material known by the trade name Porex, the function of which is essentially to stop any particulates present in the ultrafiltrate upstream of the reaction zone and, correspondingly, to retain portions of enzyme separated from the substrate in the reaction zone - preventing their dispersal towards the ultrafiltrate output line. The substrate in question is constituted, as already stated, for example, by a material such as the material known by the trade name Eupergit C, generally indicated 32.

As stated above (see formula 2 in the portion of the present description dedicated to the theoretical basis of the invention), by means of a knowledge of the proportionality constant $\Lambda$ known as the equivalent conductance, the concentration value C can be derived on the basis of the monitoring of the change in conductivity $\Delta k$ (the variation between the measurements supplied by the two probes 11, 13).

Screws, indicated 33, hold the end portions or plugs in which the connection lines 30, 32 are disposed, on the cylindrical body 34 of the reactor which contains the enzyme (urease in the case of urea determination) conjugated with the substrate particles 32. Naturally, sealing elements such as O-rings are normally inserted between the plugs and the cylindrical body.

The following known method may be used to conjugate the urease enzyme with the particles of the Eupergit C substrate.

As the first step, from 10 to 200 ml of enzyme are dissolved in 4 ml of 1M potassium phosphate buffer (pH-7.5, produced by mixing 23 ml of $KH_2PO_4$ with 77 ml of 1M $K_2HPO_4$ containing 500 ppm, that is 50 mg in 2 ml of isopropanol, ethyl diester of p-hydroxybenzoic acid as an anti-microbial agent).

The enzyme solution is then added to 1000 mg of Eupergit C in a wide-necked flask. The flask is very firmly closed and is then left at ambient temperature and without stirring for from 16 to 72 hours.

The mixture is then washed on a glass filter (porosity 2-5) with the use of a 0.1M potassium phosphate buffer (pH = 7.5) produced by mixing 170 ml of 0.1M $KH_2PO_4$ with 830 ml of $K_2HPO_4$ + 500 ppm of the ethyl ester of p-hydroxybenzoic acid. The product (urease conjugated with Eupergit C) is to be kept at +5°C after the transfer of the 0.1M phosphate buffer to which 1 mM EDTA and 1 mM reduced glutathione are added; the latter act as protectors against heavy metals.

Figures 3 to 7 show some experimental results which demonstrate the correct operation of the solution according to the invention.

In particular, the graph of Figure 3 gives the variation (the difference) between the conductivity values measured in the urea solution before and after the total enzymatic conversion of the urea into the ammonium salt $(NH_4)_2CO_3$, as a function of the concentration (in mM) in a vector fluid constituted by a 50 mM glycine buffer + 0.9% NaCl. The curve which represents the conductivity variation is the continuous line connecting the dots in the graph.

The other graph (constituted by a broken line connecting small triangles) constitutes the conductivity value detected (for the same concentration values) in a solution of the ammonium salt in the same vector.

It can be noted that the correlation between the two curves is very close (below the measurement tolerances) and demonstrates the importance of total and not only partial conversion, for the purposes of correct concentration measurement.

The factor which makes the concentration measurement method of the invention independent of any calibration operation actually lies in the complete nature of the conversion; calibration is or would be necessary, however, in methods which do not provide for total conversion but only for partial conversion with the consequent need - in finding the concentration value from the variation between the conductivity values detected before and after the conversion - for the degree of conversion (which factor depends on numerous parameters of the process).

The graph of Figure 4 is intended to show the wholly deterministic character and the degree of absolute repeatability of the measurement method according to the invention.

This graph shows the conductivity values (in mS/cm) as a function of time obtained by carrying out a complete dialysis of bovine blood to which a quantity of urea equal to 50 mmoles/l had been added with the subsequent addition after 80 min. of a second mass (bolus) of urea, again equal to 50 mmoles/l. As can be seen, the two conductivity curves have exactly the same time trace.

Figure 5 reproduces the first part of the graph of Figure 4 with the ordinate scale changed to a logarithmic scale. The graph of Figure 5 shows the exponential behaviour of the urea concentration with respect to time and its linearization gives rise to a straight line with a correlation coefficient of 0.999.

Finally, the graphs of Figures 6 and 7 relate to tests to determine the concentration of urea in blood (Figure 6) and in ultrafiltrate (Figure 7). In particular, in both of the graphs, the abscissa scale gives the data of the concentration detected by a sensor according to the invention, and the ordinate scale gives the concentration values measured by reference methods (of a photometric nature) currently used in clinical laboratories. The correlation coefficients found are 0.993 and 0.997 for the data relating to blood and to ultrafiltrate, respectively.

There is, however, reason to believe that the deviations in the correlations are to be attributed mainly to inaccuracies in the conventional monitoring methods.

The method according to the invention thus enables a determination (measurement) to be carried out on-line, in real time, obtaining measurement values which are generally much more precise and reliable than the values obtainable by conventional methods which, above all, do not permit operation on-line and/or in real time.

Naturally, the principle of the invention remaining the same, the details of construction and forms of embodiment may be varied widely with respect to those described and illustrated, without thereby departing from the scope of the present invention.

## Claims

1. A method of determining the concentration of a substance in a fluid, comprising the steps of:

    - determining (11) the electrical conductivity of the fluid containing the substance, obtaining a first conductivity value (11a),

- subjecting the substance in the fluid to enzymatic conversion into conversion products so as to bring about a change in the electrical conductivity of the fluid containing the conversion products,

- determining (13) the electrical conductivity of the fluid containing the conversion products, obtaining a second conductivity value (13a), and

- detecting (14, 15) the variation between the first (11a) and second (13a) electrical conductivity values, the variation being indicative of the concentration (C) of the substance in the fluid, characterized in that said enzymatic conversion is carried out as a total enzymatic conversion of said substance into said conversion products, and the temperature of said fluid is monitored by thermometric elements (24) to effect a temperature compensation step in obtaining said first (11d) and second (13d) conductivity values.

**2.** A method according to Claim 1, characterized in that the variation is detected as the difference between the first (11a) and second (13a) electrical conductivity values.

**3.** A method according to Claim 1 or Claim 2, characterized in that it comprises the step of directing the fluid containing the substance into a flow, and in that the determination (11) of the electrical conductivity of the fluid containing the substance, the total enzymatic conversion (12) of the substance in the fluid into the conversion products, and the determination (13) of the electrical conductivity of the fluid containing the conversion products are carried out on the flow in cascade.

**4.** A method according to any one of the preceding claims, characterized in that it comprises the step of directing the fluid containing the substance into a flow and the step of taking at least one branch flow from the flow, and in that the concentration of the substance is determined by operating on the branch flow.

**5.** A method according to any one of the preceding claims, characterized in that the substance is selected from the group constituted by urea, creatinine, glucose, barbiturate, L-arginine, penicillin and acetylcholine.

**6.** A method according to Claim 1, characterized in that the total enzymatic conversion is carried out with the use of at least one enzyme corresponding to the substance the concentration of which is to be determined.

**7.** A method according to Claim 6, characterized in that the total enzymatic conversion is carried out with the use of a plurality of enzymes.

**8.** A method according to Claim 6, characterized in that the substance is urea and the at least one enzyme is urease.

**9.** A method according to Claim 7, characterized in that the substance is creatinine, and in that the plurality of enzymes comprises creatinine amidohydrolase, creatine amidohydrolase, and urease.

**10.** A method according to Claim 6, characterized in that the substance is glucose, and in that the at least one enzyme is glucose oxidase.

**11.** A method according to Claim 7, characterized in that the substance is barbiturate and the plurality of enzymes comprises barbiturase and urease.

**12.** A method according to Claim 7, characterized in that the substance is L-arginine and the plurality of enzymes comprises arginase and urease.

**13.** A method according to Claim 6, characterized in that the substance is penicillin and the at least one enzyme is penicillinase.

**14.** A method according to Claim 6, characterized in that the substance is acetylcholine and the at least one enzyme is acetylcholinesterase.

**15.** A method according to Claim 7, characterized in that the plurality of enzymes comprises urease.

**16.** A method according to Claim 1, characterized in that the total enzymatic conversion is carried out with the use of at least one enzyme bonded covalently to a respective substrate (32).

**17.** A method according to any one of the preceding claims, characterized in that the first (11) and second (13) conductivity values are detected by determining the degree of coupling or magnetic induction between two coils (21, 22) through which the flow passes.

**18.** A system for carrying out the method according to any one of the preceding claims, characterized in that it comprises at least one flow line (20, 30, 31) through which the fluid is intended to pass and in which there are interposed in cascade

- first conductimetric means (11) for determining the electrical conductivity of the fluid containing the substance in order to obtain the first conductivity value (11a),

- an enzymatic reactor (12) packed with excess enzyme for subjecting the substance in the fluid to total enzymatic conversion into conversion products so as to bring about the change in the electrical conductivity of the fluid containing the conversion products,

- second conductimetric means (13) for determining the electrical conductivity of the fluid containing the conversion products in order to obtain the second conductivity value (13a), and

- detector means (14, 15) for detecting the variation between the first (11a) and the second (13a) electrical conductivity values, the variation being indicative of the concentration (C) of the substance in the fluid,
  wherein said first (11) and second (13) conductimetric means have associated thermometric elements (24) to monitor the temperature of said fluid to put into effect a temperature compensation step in obtaining said first (11a) and second (13a) electrical conductivity values.

**19.** A system according to Claim 18, characterized in that the detector means (14) detect the variation as the difference between the first (11a) and second (13a) electrical conductivity values.

**20.** A system according to Claim 18 or Claim 19, characterized in that the enzymatic reactor (12) contains at least one enzyme bonded covalently to a respective substrate (32).

**21.** A system according to any one of the preceding Claims 18 to 20, characterized in that each of the first (11) and second (13) conductimetric means comprises:

- a first electrical coil (21) interposed in the flow line,

- a second electrical coil (22) interposed in the flow line, and

- means (23, 23a) which are sensitive to the coupling or magnetic induction between the first coil (21) and the second coil (22).

**22.** The use of the method according to any one of Claims 1 to 17 for determining the concentration of at least one substance in a respective fluid within equipment for purifying at least one biological fluid.

**23.** Use according to Claim 22, in which the at least one biological fluid is blood.

**24.** Use according to Claim 22 or Claim 23, in which the respective fluid is ultrafiltrate derived from a haemofiltration process.

**Patentansprüche**

**1.** Verfahren zur Bestimmung der Konzentration einer Substanz im einem flüssigen Medium, wobei das Verfahren folgende Schritte enthält:

- Bestimmen (11) der elektrischen Leitfähigkeit des flüssigen Mediums, das die Substanz enthält, wobei man einen ersten Leitfähigkeitswert (11a) erhält,
- Unterwerfen der Substanz im flüssigen Medium einer enzymatischen Umwandlung in Umwandlungsprodukte, um eine Änderung der elektrischen Leitfähigkeit jenes flüssigen Mediums hervorzurufen, das die Umwandlungsprodukte enthält,

- Bestimmen (13) der elektrischen Leitfähigkeit jenes flüssigen Mediums, das die Umwandlungsprodukte enthält, wobei man einen zweiten Leitfähigkeitswert (13a) erhält, und
- Bestimmen (14, 15) der Änderung zwischen dem ersten (11a) und dem zweiten (13a) elektrischen Leitfähigkeitswert, wobei die Änderung die Konzentration (C) der Substanz im flüssigen Medium anzeigt,
dadurch gekennzeichnet, dass
die enzymatische Umwandlung als vollständige enzymatische Umwandlung der Substanz in die Umwandlungsprodukte ausgeführt wird, und dass die Temperatur des flüssigen Mediums mit Temperatur-Messelementen (24) überwacht wird, um beim Erhalten des ersten (11a) und des zweiten (13a) Leitfähigkeitswerts einen Schritt für die Temperaturkompensation auszuführen.

2. Verfahren gemäß Anspruch 1, dadurch gekennzeichnet, dass die Änderung als die Differenz zwischen dem ersten (11a) und dem zweiten (13a) elektrischen Leitfähigkeitwert bestimmt wird.

3. Verfahren gemäß Anspruch 1 oder Anspruch 2, dadurch gekennzeichnet, dass das Verfahren einen Schritt enthält, um jenes flüssige Medium, das die Substanz enthält, in eine Strömung zu bringen, und dass die Bestimmung (11) der elektrischen Leitfähigkeit jenes flüssigen Mediums, das die Substanz enthält, die vollständige enzymatische Umwandlung (12) der Substanz im flüssigen Medium in die Umwandlungsprodukte sowie die Bestimmung (13) der elektrischen Leitfähigkeit des flüssigen Mediums, das die Umwandlungsprodukte enthält, in der Strömung in Kaskade ausgeführt werden.

4. Verfahren gemäß irgendeinem der bisherigen Ansprüche, dadurch gekennzeichnet, dass das Verfahren einen Schritt enthält, um das flüssige Medium, das die Substanz enthält, in eine Strömung zu bringen, sowie einen Schritt enthält, um zumindest eine Nebenströmung von der Strömung abzuleiten, und dass die Konzentration der Substanz dadurch bestimmt wird, dass die Nebenströmung bearbeitet wird.

5. Verfahren gemäß irgendeinem der bisherigen Ansprüche, dadurch gekennzeichnet, dass die Substanz aus einer Gruppe ausgewählt wird, die von Harnstoff, Kreatinin, Glucose, Barbiturat, L-Arginin, Penicillin und Acetylcholin gebildet wird.

6. Verfahren gemäß Anspruch 1, dadurch gekennzeichnet, dass die vollständige enzymatische Umwandlung unter Verwendung von zumindest einem Enzym durchgeführt wird, das jener Substanz entspricht, deren Konzentration bestimmt werden soll.

7. Verfahren gemäß Anspruch 6, dadurch gekennzeichnet, dass die vollständige enzymatische Umwandlung unter Verwendung einer Mehrzahl von Enzymen durchgeführt wird.

8. Verfahren gemäß Anspruch 6, dadurch gekennzeichnet, dass die Substanz Harnstoff und das zumindest ein Enzym Urease ist.

9. Verfahren gemäß Anspruch 7, dadurch gekennzeichnet, dass die Substanz Kreatinin ist, und dass die Mehrzahl von Enzymen Kreatininamidohydrolase, Kreatinamidohydrolase und Urease umfasst.

10. Verfahren gemäß Anspruch 6, dadurch gekennzeichnet, dass die Substanz Glucose ist, und dass das zumindest eine Enzym Glucoseoxidase ist.

11. Verfahren gemäß Anspruch 7, dadurch gekennzeichnet, dass die Substanz Barbiturat ist und die Mehrzahl von Enzymen Barbiturase und Urease umfasst.

12. Verfahren gemäß Anspruch 7, dadurch gekennzeichnet, dass die Substanz L-Arginin ist und die Mehrzahl von Enzymen Arginase und Urease umfasst.

13. Verfahren gemäß Anspruch 6, dadurch gekennzeichnet, dass die Substanz Penicillin ist und dass zumindest ein Enzym Penicillinase ist.

14. Verfahren gemäß Anspruch 6, dadurch gekennzeichnet, dass die Substanz Acetylcholin und das zumindest ein Enzym Acetylcholinesterase ist.

15. Verfahren gemäß Anspruch 7, dadurch gekennzeichnet, dass die Mehrzahl von Enzymen Urease umfasst.

16. Verfahren gemäß Anspruch 1, dadurch gekennzeichnet, dass die vollständige enzymatische Umwandlung unter Verwendung von zumindest einem Enzym durchgeführt wird, das kovalent an ein entsprechendes Substrat (32) gebunden ist.

17. Verfahren gemäß irgendeinem der bisherigen Ansprüche, dadurch gekennzeichnet, dass der erste (11) und der zweite (13) Leitfähigkeitswert dadurch bestimmt werden, dass der Kopplungsgrad oder die magnetische Induktion zwischen zwei Spulen (21, 22) bestimmt werden, durch die die Strömung fließt.

18. System, um das Verfahren gemäß irgendeinem der bisherigen Ansprüche auszuführen, dadurch gekennzeichnet, dass das System zumindest eine Strömungsleitung (20, 30, 31) enthält, durch die das flüssige Medium fließen soll und in der in Kaskade geschaltet sind:

- eine erste Leitfähigkeits-Messeinrichtung (11), um die elektrische Leitfähigkeit des flüssigen Mediums zu bestimmen, das die Substanz enthält, um den ersten Leitfähigkeitswert (11a) zu erhalten,
- ein Enzym-Reaktionsgefäß (12), das mit einem Überschuss an Enzym gepackt ist, um die Substanz in dem flüssigen Medium einer vollständigen enzymatischen Umwandlung in Umwandlungsprodukte zu unterwerfen, um die Änderung in der elektrischen Leitfähigkeit des flüssigen Mediums hervorzurufen, das die Umwandlungsprodukte enthält,
- eine zweite Leitfähigkeits-Messeinrichtung (13), um die elektrische Leitfähigkeit des flüssigen Mediums zu bestimmen, das die Umwandlungsprodukte enthält, um den zweiten Leitfähigkeitswert (13a) zu erhalten, und
- eine Messeinrichtung (14, 15), um die Änderung zwischen dem ersten (11a) und dem zweiten (13a) elektrischen Leitfähigkeitswert zu bestimmen, wobei die Änderung die Konzentration (C) der Substanz im flüssigen Medium anzeigt,

  wobei der ersten (11) und der zweiten (13) Leitfähigkeits-Messeinrichtung Temperatur-Messelemente (24) zugeordnet sind, um die Temperatur des flüssigen Mediums zu überwachen, um einen Schritt zur Temperaturkompensation auszuführen, wenn man den ersten (11a) und den zweiten (13a) elektrischen Leitfähigkeitswert erhält.

19. System gemäß Anspruch 18, dadurch gekennzeichnet, dass die Messeinrichtung (14) die Änderung als die Differenz zwischen dem ersten (11a) und dem zweiten (13a) elektrischen Leitfähigkeitswert bestimmt.

20. System gemäß Anspruch 18 oder Anspruch 19, dadurch gekennzeichnet, dass das Enzym-Reaktionsgefäß (12) zumindest ein Enzym enthält, das kovalent an ein entsprechendes Substrat (32) gebunden ist.

21. System gemäß irgendeinem der bisherigen Ansprüche 18 bis 20, dadurch gekennzeichnet, dass sowohl die erste (11) als auch die zweite (13) Leitfähigkeits-Messeinrichtung enthält:

- eine erste elektrische Spule (21), die in der Strömungsleitung angeordnet ist,
- eine zweite elektrische Spule (22), die in der Strömungsleitung angeordnet ist, und
- eine Einrichtung (23, 23a), die auf die Kopplung oder die magnetische Induktion zwischen der ersten Spule (21) und der zweiten Spule (22) anspricht.

22. Verwendung des Verfahrens gemäß irgendeinem der Ansprüche 1 bis 17, um die Konzentration von zumindest einer Substanz in einem entsprechenden flüssigen Medium in einem Gerät für die Reinigung von zumindest einem biologischen flüssigen Medium zu bestimmen.

23. Verwendung gemäß Anspruch 22, wobei das zumindest eine biologische flüssige Medium Blut ist.

24. Verwendung gemäß Anspruch 22 oder Anspruch 23, wobei das entsprechende flüssige Medium ein Ultrafiltrat aus einem Hämofiltrationsverfahren ist.

## Revendications

1. Procédé pour la détermination de la concentration d'une substance dans un fluide, comprenant les étapes consistant:

- à déterminer (11) la conductivité électrique du fluide contenant la substance, en obtenant une première valeur (11a) de la conductivité,

- à soumettre la substance présente dans le fluide à une conversion enzymatique en des produits de conversion de façon à provoquer un changement au niveau de la conductivité électrique du fluide contenant les produits de conversion,
- à déterminer (13) la conductivité électrique du fluide contenant les produits de conversion, en obtenant une seconde valeur (13a) de la conductivité, et
- à détecter (14, 15) la variation entre la première valeur (11a) et la seconde valeur (13a) de la conductivité électrique, cette variation indiquant la concentration (C) de la substance dans le fluide,

caractérisé en ce que ladite conversion enzymatique est réalisée par une conversion enzymatique totale de ladite substance en lesdits produits de conversion, et la température dudit fluide est contrôlée par des éléments thermométriques (24) pour effectuer une étape de compensation de température lors de l'obtention desdites première (11a) et seconde (13a) valeurs de la conductivité.

**2.** Procédé selon la revendication 1, caractérisé en ce que la variation est détectée par la différence entre la première valeur (11a) et la seconde valeur (13a) de la conductivité électrique.

**3.** Procédé selon la revendication 1 ou la revendication 2, caractérisé en ce qu'il comprend l'étape consistant à diriger le fluide contenant la substance dans un circuit, et en ce que la détermination (11) de la conductivité électrique du fluide contenant la substance, la conversion enzymatique totale (12) de la substance présente dans le fluide en les produits de conversion, et la détermination (13) de la conductivité électrique du fluide contenant les produits de conversion, sont réalisées dans le circuit, en cascade.

**4.** Procédé selon l'une quelconque des revendications précédentes, caractérisé en ce qu'il comprend l'étape consistant à diriger le fluide contenant la substance dans un circuit, et l'étape consistant à prélever dans le circuit au moins un flux dérivé, et en ce que la concentration de la substance est déterminée en exploitant le flux dérivé.

**5.** Procédé selon l'une quelconque des revendications précédentes, caractérisé en ce que la substance est choisie dans le groupe constitué de l'urée, de la créatinine, du glucose, du barbiturate, de la L-arginine, de la pénicilline et de l'acétylcholine.

**6.** Procédé selon la revendication 1, caractérisé en ce que la conversion enzymatique totale est réalisée en utilisant au moins une enzyme correspondant à la substance dont la concentration doit être déterminée.

**7.** Procédé selon la revendication 6, caractérisé en ce que la conversion enzymatique totale est réalisée en utilisant plusieurs enzymes.

**8.** Procédé selon la revendication 6, caractérisé en ce que la substance est de l'urée et qu'au moins l'une des enzymes est l'uréase.

**9.** Procédé selon la revendication 7, caractérisé en ce que la substance est de la créatinine, et en ce que les enzymes comprennent la créatinine-amidohydrolase, la créatine-amidohydrolase, et l'uréase.

**10.** Procédé selon la revendication 6, caractérisé en ce que la substance est du glucose, et en ce qu'au moins l'une des enzymes est la glucose-oxydase.

**11.** Procédé selon la revendication 7, caractérisé en ce que la substance est du barbiturate, et les enzymes comprennent la barbiturase et l'uréase.

**12.** Procédé selon la revendication 7, caractérisé en ce que la substance est de la L-arginine, et les enzymes comprennent l'arginase et l'uréase.

**13.** Procédé selon la revendication 6, caractérisé en ce que la substance est de la pénicilline, et au moins l'une des enzymes est la pénicillinase.

**14.** Procédé selon la revendication 6, caractérisé en ce que la substance est de l'acétylcholine, et au moins l'une des enzymes est l'acétylcholinestérase.

**15.** Procédé selon la revendication 7, caractérisé en ce que les enzymes comprennent l'uréase.

**16.** Procédé selon la revendication 1, caractérisé en ce que la conversion enzymatique totale est réalisée en utilisant au moins une enzyme fixée par covalence à son substrat respectif (32).

**17.** Procédé selon l'une quelconque des revendications précédentes, caractérisé en ce que la première (11) et la seconde (13) valeurs de la conductivité sont détectées en déterminant le degré de couplage ou d'induction magnétique entre deux bobines (21, 22) à travers lesquelles le flux s'écoule.

**18.** Système permettant de mettre en oeuvre le procédé selon l'une quelconque des revendications précédentes, caractérisé en ce qu'il comprend au moins un conduit d'écoulement (20, 30, 31) à travers lequel le fluide est destiné à passer et dans lequel on a intercalé en cascade

- un premier dispositif conductimétrique (11) pour déterminer la conductivité électrique du fluide contenant la substance, afin d'obtenir la première valeur (11a) de la conductivité,
- un réacteur enzymatique (12) garni avec un excès d'enzyme pour soumettre la substance présente dans le fluide à une conversion enzymatique totale en des produits de conversion, de façon à provoquer un changement de la conductivité électrique du fluide contenant les produits de conversion,
- un second dispositif conductimétrique (13) pour déterminer la conductivité électrique du fluide contenant les produits de conversion afin d'obtenir la seconde valeur (13a) de la conductivité, et
- des moyens de détection (14, 15) pour détecter la variation entre la première (11a) et la seconde (13a) valeur de la conductivité électrique, la variation indiquant la concentration (C) de la substance dans le fluide, dans lequel lesdits premier (11) et second (13) dispositifs conductimétriques sont associés à des éléments thermométriques (24) pour contrôler la température dudit fluide pour mettre en oeuvre une étape de compensation de température lors de l'obtention desdites première (11a) et seconde (13a) valeurs de la conductivité électrique.

**19.** Système selon la revendication 18, caractérisé en ce que les moyens de détection (14) détectent la variation sous la forme de la différence entre la première valeur (11a) et la seconde valeur (13a) de la conductivité électrique.

**20.** Système selon la revendication 18 ou la revendication 19, caractérisé en ce que le réacteur enzymatique (12) contient au moins une enzyme fixée par covalence à son substrat respectif (32).

**21.** Système selon l'une quelconque des revendications 18 à 20 précédentes, caractérisé en ce que chacun du premier (11) et du second (13) dispositif conductimétrique comprend:

- une première bobine électrique (21) intercalée dans le conduit d'écoulement,
- une seconde bobine électrique (22) intercalée dans le conduit d'écoulement, et
- des dispositifs (23, 23a) qui sont sensibles au couplage ou à l'induction magnétique entre la première bobine (21) et le seconde bobine (22).

**22.** Utilisation du procédé selon l'une quelconque des revendications 1 à 17, pour déterminer la concentration d'au moins une substance dans son fluide respectif au sein d'un équipement destiné à purifier au moins un fluide biologique.

**23.** Utilisation selon la revendication 22, dans laquelle au moins l'un des fluides biologiques est du sang.

**24.** Utilisation selon la revendication 22 ou la revendication 23, dans laquelle le fluide respectif est dérivé par ultrafiltration d'un procédé d'hémofiltration.

FIG. 1

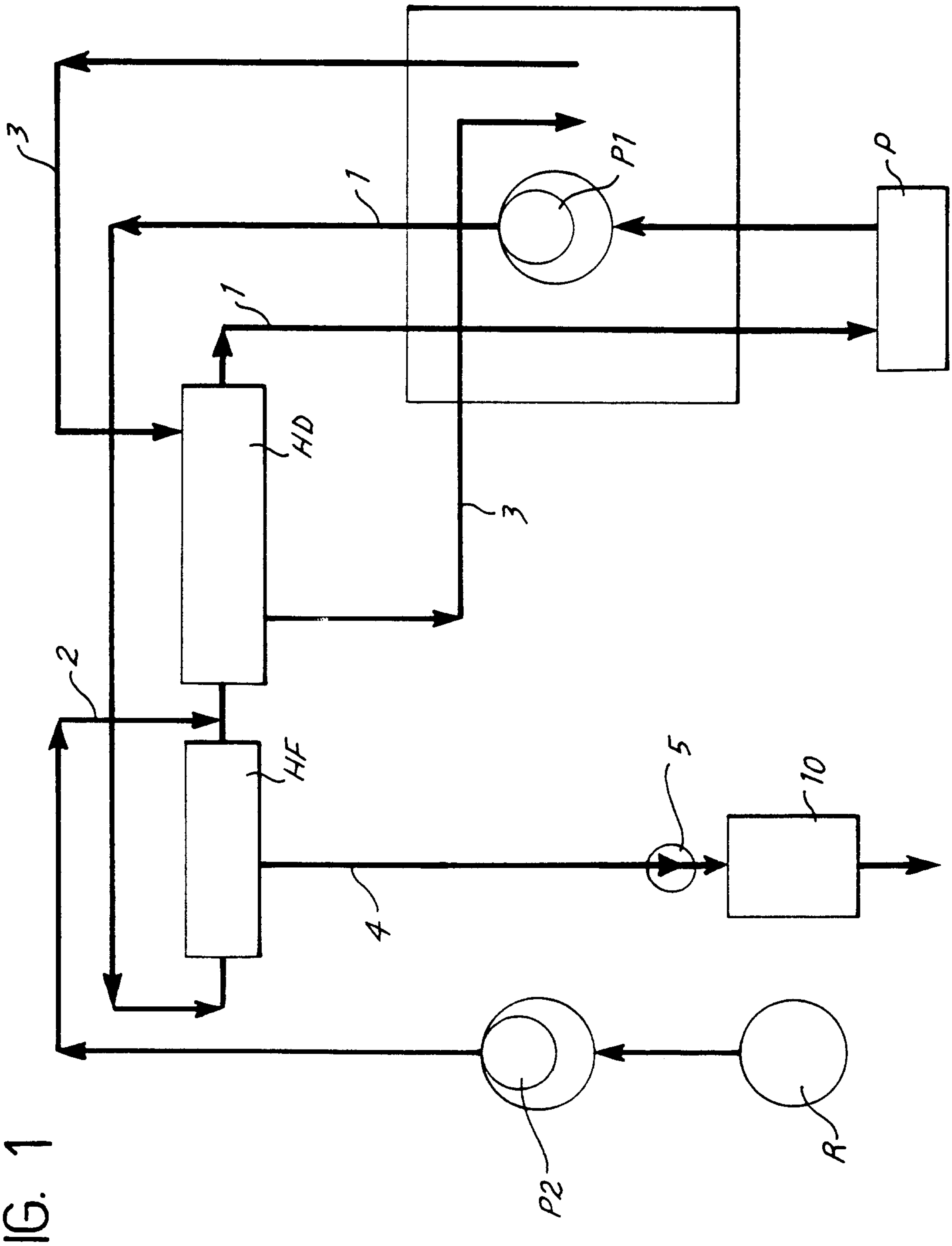

FIG. 2

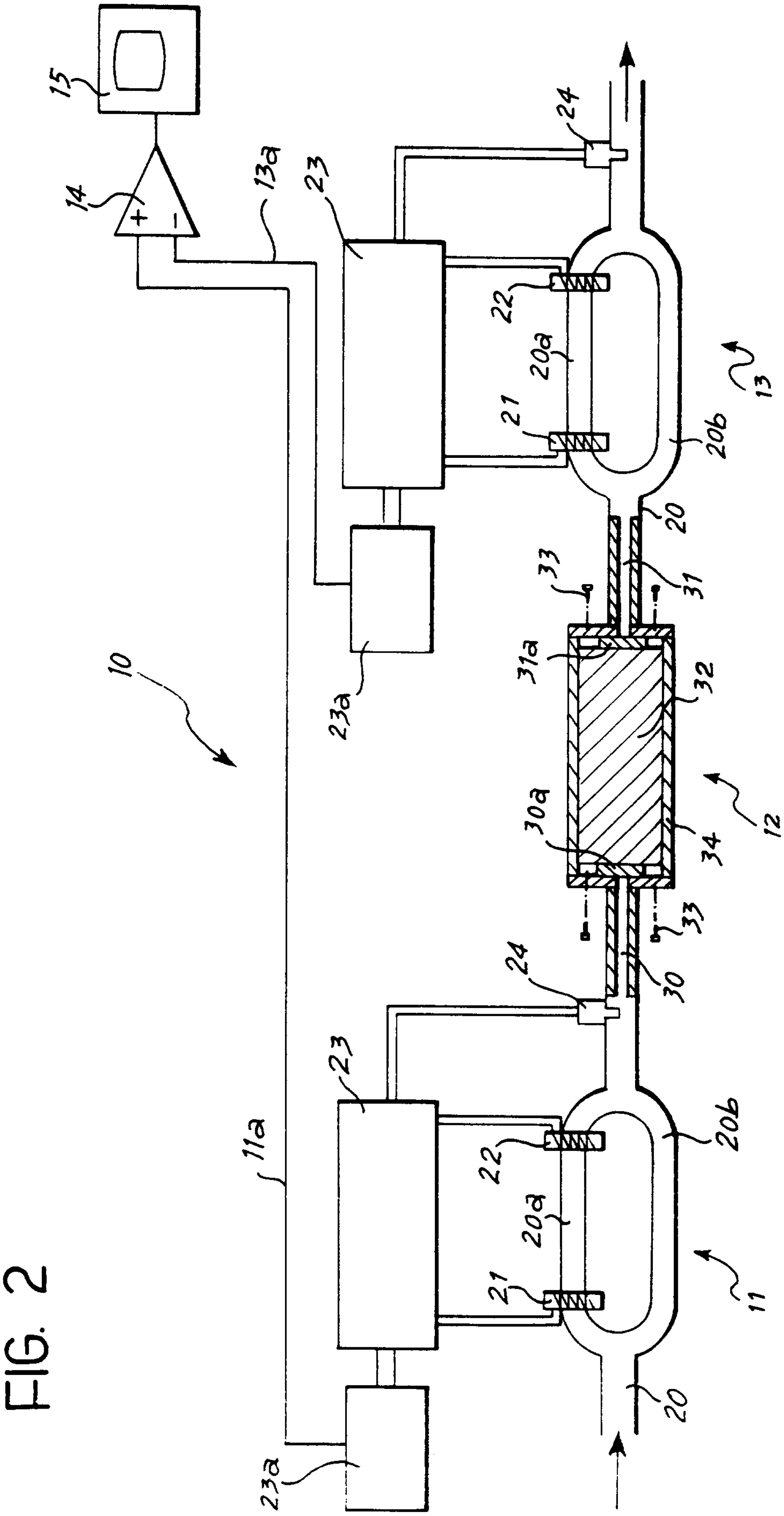
15
14
+
-
13a
23
24
22
20a
21
20b
13
20
33
31
31a
32
23a
10
12
34
30a
33
30
24
23
20b
11a
22
20a
11
21
20
23a

FIG. 3

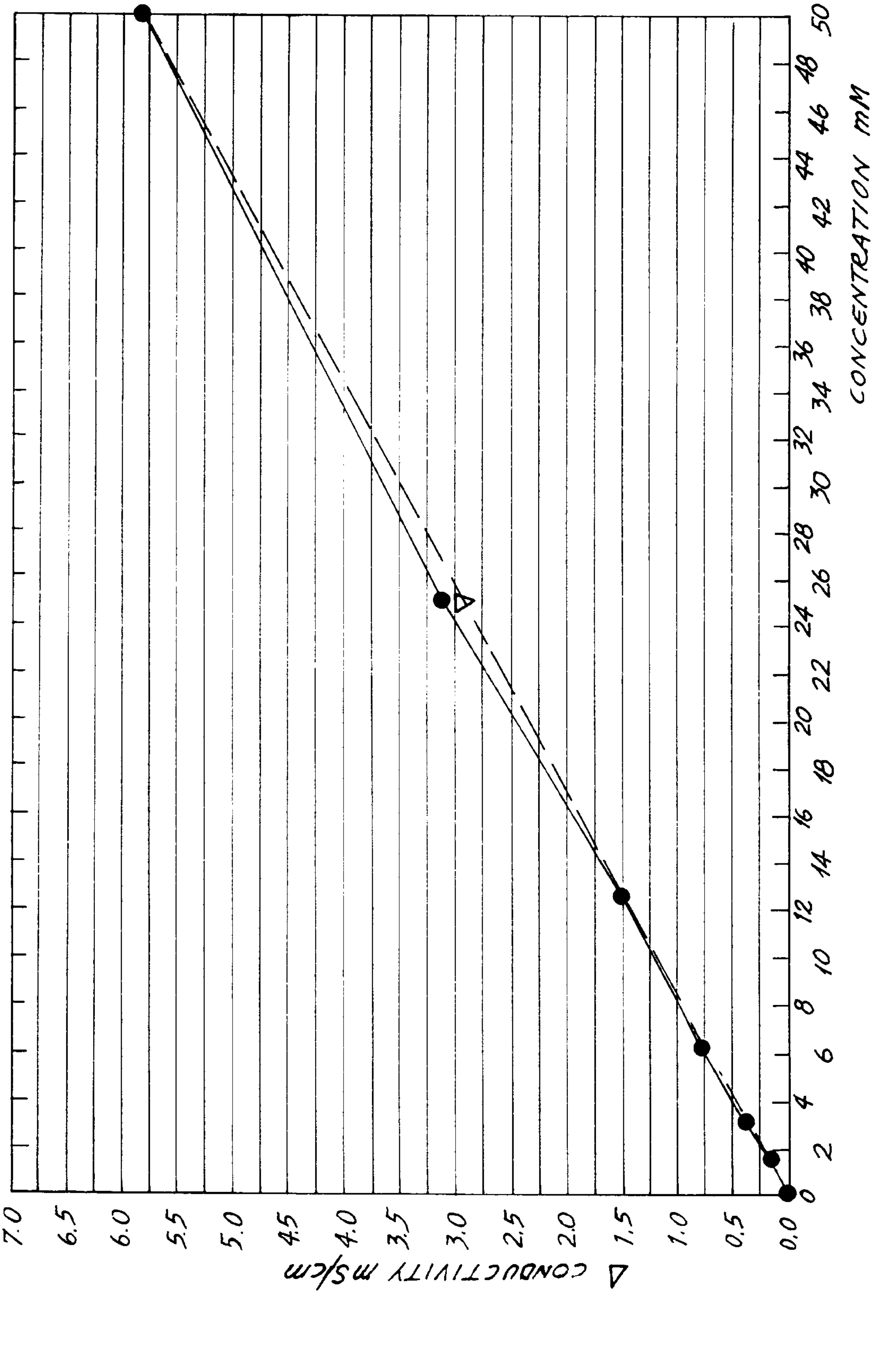
CONCENTRATION mM
0
2
4
6
8
10
12
14
16
18
20
22
24
26
28
30
32
34
36
38
40
42
44
46
48
50
Δ CONDUCTIVITY mS/cm
0.0
0.5
1.0
1.5
2.0
2.5
3.0
3.5
4.0
4.5
5.0
5.5
6.0
6.5
7.0

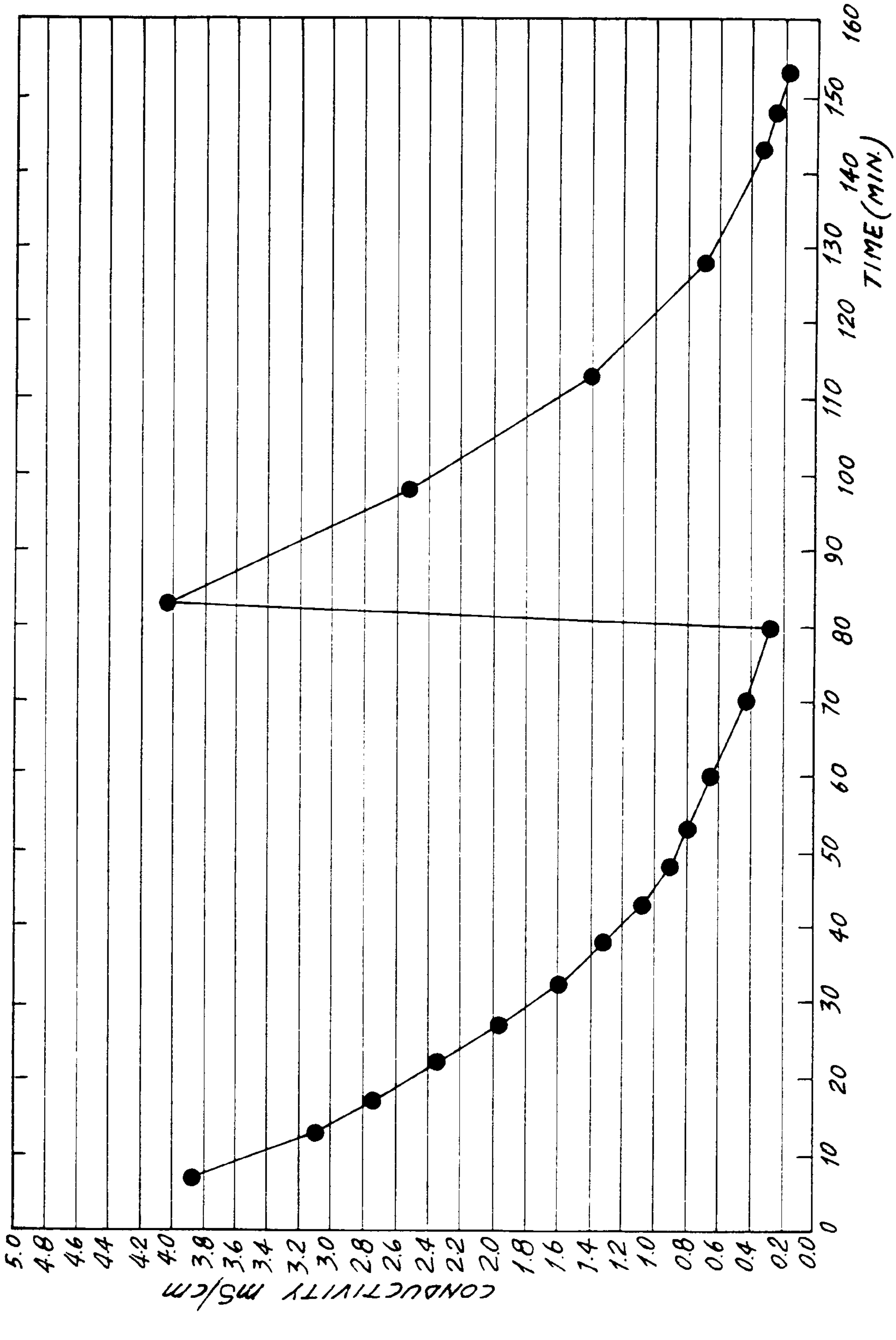
CONDUCTIVITY mS/cm
5.0
4.8
4.6
4.4
4.2
4.0
3.8
3.6
3.4
3.2
3.0
2.8
2.6
2.4
2.2
2.0
1.8
1.6
1.4
1.2
1.0
0.8
0.6
0.4
0.2
0.0
TIME (MIN.)
0
10
20
30
40
50
60
70
80
90
100
110
120
130
140
150
160

FIG. 4

FIG. 5

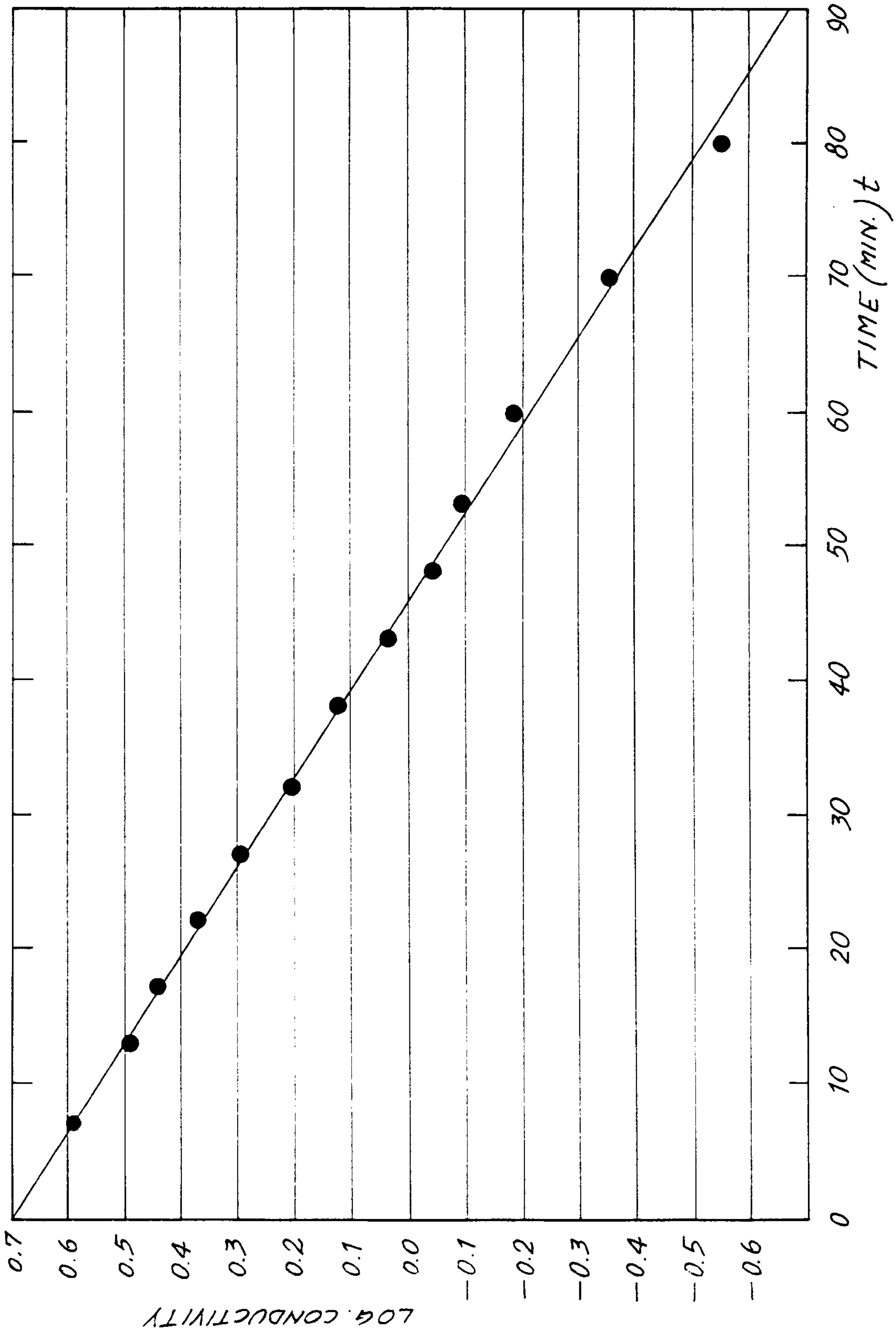
LOG. CONDUCTIVITY
0.7
0.6
0.5
0.4
0.3
0.2
0.1
0.0
−0.1
−0.2
−0.3
−0.4
−0.5
−0.6
TIME (MIN.) t
0
10
20
30
40
50
60
70
80
90

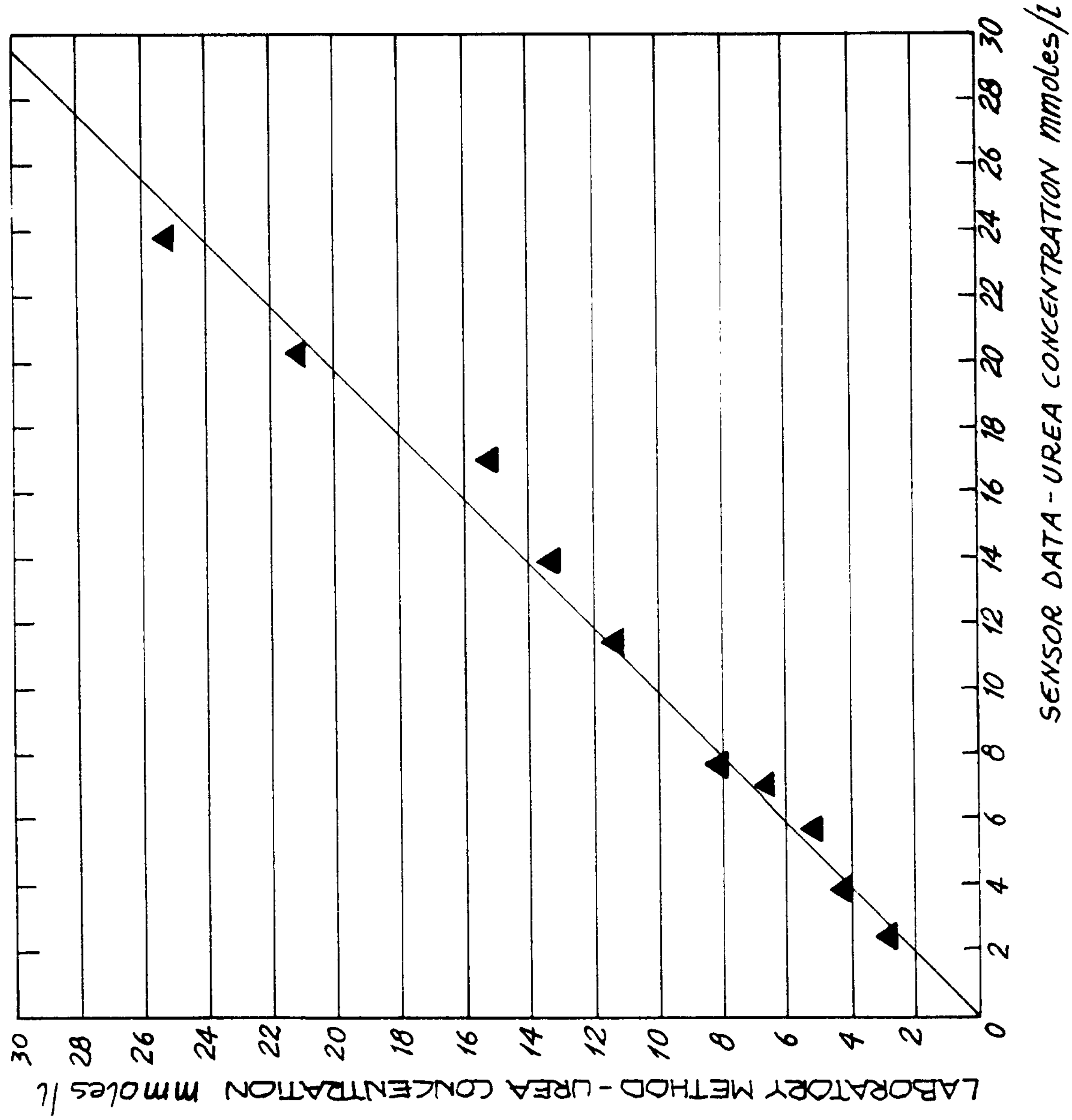
SENSOR DATA - UREA CONCENTRATION mmoles/l
LABORATORY METHOD - UREA CONCENTRATION mmoles/l
0 2 4 6 8 10 12 14 16 18 20 22 24 26 28 30
2 4 6 8 10 12 14 16 18 20 22 24 26 28 30

FIG. 6

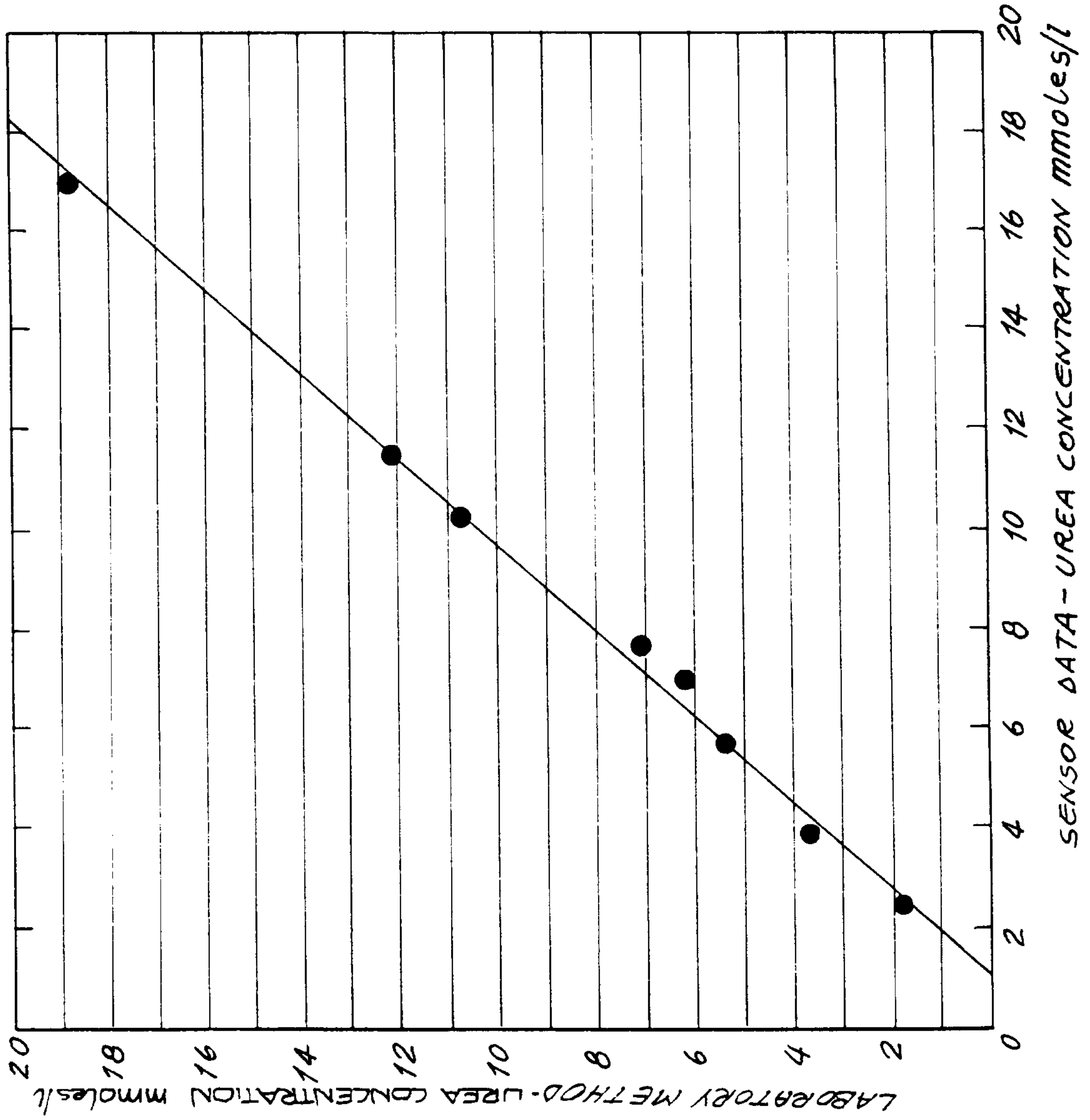
SENSOR DATA-UREA CONCENTRATION mmoles/l
LABORATORY METHOD-UREA CONCENTRATION mmoles/l
0 2 4 6 8 10 12 14 16 18 20

FIG. 7